# EUROPEAN PATENT APPLICATION

(11) **EP 2 312 303 A1**
(43) Date of publication of application: **20.04.2011**
(21) Application number: 09172765.1
(22) Date of filing: 12.10.2009
(51) Int. Cl.: G01N 24/08, G01R 33/44, G01R 33/48, G01R 33/54, A61B 8/00, A61B 8/13, A61N 7/02

(54) **Magnetic resonance imaging system and method for detecting a gas bubble**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

A magnetic resonance imaging system (100) for detecting a gas bubble (124, 148, 304, 306, 404, 406) within an imaging volume (108), the magnetic resonance imaging system comprising:
- a magnet (102) adapted for generating a magnetic field for orientating the magnetic spins of nuclei of a subject (104) located within the imaging volume;
- a radio frequency system (110, 112) adapted for acquiring magnetic resonance data (160, 164), wherein the radio frequency system comprises a radio frequency transceiver (112) and a radio frequency coil (110);
- a magnetic field gradient coil (114) adapted for spatial encoding of the magnetic spins of nuclei within the imaging volume;
- a magnetic field gradient coil power supply (116) adapted for supplying current to the magnetic field gradient coil; and
- a computer system (132) adapted for constructing images from the magnetic resonance imaging data and for controlling the operation of the magnetic resonance imaging system, wherein the computer system is adapted for detecting the gas bubble within the imaging volume using a magnetic resonance image (162, 166, 300, 400) constructed from the magnetic resonance imaging data.

## Description

### TECHNICAL FIELD

The invention relates to image processing, in particular the identification of gas bubbles in magnetic resonance images.

### BACKGROUND OF THE INVENTION

Ultrasound from a focused ultrasound transducer can be used to selectively treat regions within the interior of the body. Ultrasonic waves are transmitted as high energy mechanical vibrations. These vibrations induce tissue heating as they are damped, and as high-intensity fields they can also lead to cavitation. Both tissue heating and cavitation can be used to intentionally destroy tissue in a clinical setting. However, heating tissue with ultrasound is easier to control than cavitation and cavitation outside intended target may result in unintended thermal lesions, e.g. at patient skin. Ultrasonic treatments can be used to ablate tissue and to kill regions of tissue tumors selectively. This technique has been applied to the treatment of uterine fibroids, and has reduced the need for hysterectomy procedures often requiring long recovery and hospitalization.

To selectively treat tissue, a focused ultrasound transducer can be used to focus the ultrasound on a particular treatment volume. The transducer is able to transmit ultrasound , and is typically mounted within a medium, such as degassed water. Actuators are then used to adjust the position of the ultrasound transducer and thereby adjust the region of a subject that is being treated with ultrasound. Electric focusing may be used to steer or shape the ultrasound field to ablate larger tissue volumes.

Standard medical imaging techniques are commonly used to plan the ultrasound procedure and can also be used to guide the treatment. Magnetic Resonance Imaging (MRI), Computed Tomography (CT), and ultrasonic imaging have been used for the planning and guiding of ultrasonic treatments. Focused ultrasound transducers typically have a limited range over which they can be actuated, so the patient must be positioned properly relative to the ultrasound system. Ultrasound is not able to be transmitted to the body through air, so an ultrasound coupling medium such as ultrasonic gel, an ultrasonic gel pad, and/or degassed water is used to transmit the ultrasound from the ultrasound system to the surface of a subject. Typically a membrane such as Mylar is used to form a boundary between the ultrasound transducer bath and the medium used to conduct ultrasound to the subject.

### SUMMARY OF THE INVENTION

The invention provides for a magnetic resonance imaging system, a computer program product and a computer implemented method in the independent claims. Embodiments are given in the depending claims.

A difficulty with ultrasound procedures is that when the subject is acoustically coupled to the ultrasound system with an ultrasound coupling medium there may be gas bubbles trapped between the surface of the subject and the ultrasound coupling medium, e.g. at gel pad - skin interface. If high intensity focused ultrasound is applied to a region in which there is a bubble or bubbles significantly larger than the wavelength, ultrasound can be reflected back to the transmitting transducer inducing transducer damage or reducing transducer lifespan. If high intensity focused ultrasound is applied to a region in which there is/are small gas bubble/bubbles, ultrasound can induce cavitation in the bubble or bubbles causing damage or burning to the surface of the subject in contact with the bubble. It is therefore beneficial to identify gas bubbles before high intensity ultrasound is applied to a subject. Embodiments of the invention may provide a solution to this technical problem by using image processing to identify a gas bubble in a magnetic resonance image. In some embodiments a gas bubble sensitive pulse sequence may be used for acquiring the magnetic resonance imaging data.

The invention provides for a magnetic resonance imaging system for detecting a gas bubble within an imaging volume. The invention may also be used for detecting multiple gas bubbles. The magnetic resonance imaging system comprises a magnet adapted for generating a magnetic field for orienting the magnetic spins of nuclei of a subject located within the imaging volume. The magnet may be a superconducting magnet, a permanent magnet, an electromagnet or a combination of any of the previous three types of magnets. The magnetic resonance imaging system further comprises a radio frequency system adapted for acquiring magnetic resonance data. Magnetic resonance data as used herein is data acquired by a magnetic resonance imaging system from a subject within an imaging volume of the magnetic resonance imaging system.

The magnetic resonance imaging data comprises a representation or recording of radio signals received from nuclei of the subject. Magnetic resonance imaging data may be used to construct images or visualizations of the imaging region of the subject. The radio signals are received predominantly from the imaging region. However, magnetic resonance images are constructed using Fourier transformations of the magnetic resonance imaging data. As a result, nuclei outside of the imaging volume may contribute to the magnetic resonance imaging data and hence any image or visualization which is constructed.

The radio frequency system comprises a radio frequency transceiver and a radio frequency coil. The radio frequency coil may be a single coil capable of transmitting and receiving radio signals, or the radio frequency coil may consist of a transmit coil and a receive coil. Similarly the radio frequency transceiver may be a transceiver, or it may be separate transmit and receive radio devices. The magnetic resonance imaging system further comprises a magnetic field gradient coil adapted for spatial encoding of the magnetic spins within the imaging volume. The magnetic resonance imaging system further comprises a magnetic field gradient coil power supply adapted for supplying current to the magnetic field gradient coil.

The magnetic resonance imaging system further comprises a computer system adapted for constructing images from the magnetic resonance imaging data and for controlling the operation of the magnetic resonance imaging system. The computer system is adapted for detecting a gas bubble within the imaging volume using magnetic resonance imaging constructed from the magnetic resonance imaging data. The detection of the gas bubble within the imaging volume is beneficial because the knowledge and location of the gas bubble may be used for treatment planning or diagnosis.

In another embodiment, the computer system is adapted for detecting the gas bubble within the imaging volume by accessing the magnetic resonance image. The computer system is further adapted for detecting the gas bubble by performing the step of creating a subtracted image by subtracting the magnetic resonance image from the trend detected image. The subtracted image is created by subtracting the magnetic resonance imaging image from the trend detected image.

The computer system is further adapted for detecting the gas bubble within the imaging volume by performing the step of creating a binary image by applying a threshold to the subtracted image. The computer system is further adapted for detecting the gas bubble within the imaging volume by performing the step of creating an output image by median filtering the binary image. As used herein a median filter is a filter, which removes speckel noise and salt and pepper noise from an image by calculating the median value of a pixel's neighbors. Median filters are effective in removing noise while preserving edges. As used herein a low pass filter of an image is a spatial filter. A low pass filter or low pass filtering of the image performs the step of removing objects below a certain size.

In another embodiment, the magnetic resonance imaging system is adapted for acquiring magnetic resonance imaging data with a gas bubble sensitive pulse sequence. The gas bubble may be detected using a gas bubble sensitive magnetic resonance pulse sequence. A pulse sequence as used herein is the sequence of operations performed by a magnetic resonance imaging system for acquiring magnetic resonance imaging data. For instance a pulse sequence contains instructions for the voltage applied to the magnetic resonance gradient coils as a function of time as well as the radio frequency signals applied to the radio frequency coil as a function of time. An example of a gas bubble sensitive sequence is a gradient echo pulse sequence where: the time to echo (TE) is 15 milliseconds, the flip angle (FA) is 60 degrees, a 1.11 mm by 1.11 mm by 2.5 mm voxel size, with maximum reconstruction resolution. Many pulse sequences are used to image the density of protons or hydrogen atoms. As the density of hydrogen atoms in a gas bubble is much lower than in a liquid such as water, many different pulse sequences are able to be used for acquiring magnetic resonance imaging data which can be used to detect gas bubbles.

In another embodiment, the gas bubble sensitive pulse sequence is a gradient echo pulse sequence. The gradient echo sequence has an echo time between 5 and 25 milliseconds, preferably between 10 and 20 milliseconds. The gradient echo sequence has a resolution of between 0.8 and 1.5 millimeters, preferably between 1 and 1.25 millimeters. At resolutions below 1 millimeter it was found to be beneficial to compensate for an increasing signal to noise ratio. Also below resolutions below 1 millimeter it was found that the number of bubbles was over estimated. At resolutions above 1.25 millimeters it was found that the number of bubbles was over estimated. The gradient echo sequence has a slice thickness of between 1 and 4 millimeters, preferably between 2 and 3 millimeters.

The gradient echo sequence has a flip angle between 10 and 80 degrees, preferably between 50 and 70 degrees. Adjusting the reconstruction resolution of the magnetic resonance image may be used to improve the quality of the bubble detection. A two or three dimensional gradient pulse sequence may be used. If a two dimensional gradient pulse sequence is used for detecting gas bubble visibility between the subject and the ultrasound window functions well when a stack of slices that is parallel with the ultrasound window. For instance, if the ultrasound window is installed in a subject support that functions as a bed a subject would then lie on the bed. A stack of images acquired in the coronal plane of the subject could be used by the magnetic resonance imaging system to detect bubbles between the subject and the ultrasound window.

In another embodiment the magnetic resonance imaging system further comprises an ultrasound system for generating ultrasonic waves. The ultrasound system comprises an ultrasound window. The ultrasound window is adapted for receiving the subject. The ultrasound window may be a window that comes in direct contact with the subject, or the ultrasound window may form a contact with the subject by using an ultrasound coupling medium. An ultrasound coupling medium is a medium which is adapted for transmitting ultrasonic waves and is able to conform to the shape of a surface. Examples of an ultrasound coupling medium would be a gel pad or ultrasound coupling gel, or water.

In another embodiment the magnetic resonance imaging system is adapted to detect the gas bubble between the ultrasound window and the subject. This embodiment is particularly advantageous, because the gas bubble affects the propagation of ultrasound. The gas bubble may scatter the ultrasound, or the ultrasound may cause cavitation of the gas bubble.

In another embodiment the ultrasound system is a high intensity focused ultrasound system. This embodiment is particularly advantageous, because high intensity ultrasound can cause the cavitation of the gas bubble. The detection of the gas bubble allows the accidental damage to a subject or portion of a subject by the cavitation of gas bubbles.

In another embodiment the computer system is adapted for calculating a path of the ultrasonic waves. The path of the ultrasonic waves may be calculated using a ray tracing technique. The computer system is adapted for calculating the attenuation of the ultrasonic waves along the path by detected by the gas bubble. The attenuation of the ultrasonic waves may be calculated by estimating the cavitation or oscillation of the radius of the gas bubble or it may be accomplished using a lookup table that uses experimental data. The magnetic resonance imaging system is further adapted for signaling an operator if the attenuation of the ultrasonic waves is above a predetermined attenuation threshold. This embodiment is advantageous because it allows an operator to reposition a subject if the gas bubble will cause too large an attenuation or a reflection of the ultrasound. The operator can be signaled in a variety of ways. There could be an audible signal, there could be a signal on a computer display or monitor, or there could be a signal indicator such as a light on a control panel which could indicate the attenuation will be too great.

In another embodiment the ultrasound system is adapted for adjusting the path of the ultrasound waves if the predetermined attenuation threshold is exceeded. This embodiment is advantageous because the use of ultrasound waves without repositioning the subject in order to remove the gas bubble from the path of the ultrasound waves. The path of the ultrasound may be accomplished in several different ways. The ultrasound system may have an ultrasound transducer which can be mechanically moved. In this case the ultrasound system may be repositioned. In other embodiments the ultrasound system may have an ultrasound transducer that has multiple ultrasound transducer elements. The individual elements may have a supply of electrical power such that the phase and amplitude of ultrasound generated by the ultrasound transducer elements may be altered. This allows the choosing of ultrasound transducer elements such that the ultrasound follows a path which is not attenuated by the detected gas bubble.

In another embodiment the computer system comprises a display. The display is adapted for displaying the output image. The output image may be displayed by itself, or it may be displayed on top of or superimposed on a magnetic resonance imaging image. The output image may also be displayed such that it is adapted for attracting the attention of an operator. For instance bright colors such as red, yellow or orange could be used to indicate the location of a bubble. Flashing images superimposed on a magnetic resonance imaging image showing anatomy could be used to emphasize the location of the detected gas bubble.

In another aspect the invention provides for a computer program product comprising machine executable instructions for execution by a computer system for performing the step of accessing a magnetic resonance image constructed from magnetic resonance imaging data of an imaging volume. The magnetic resonance image may be stored such as on a hard drive or other computer storage medium or it may be located within the random access memory of the computer system. In other words the magnetic resonance image could have been previously acquired or the process of detecting gas bubbles may be commenced as soon as a magnetic resonance image is constructed from the magnetic resonance imaging data. A computer system as used herein is a machine adapted for performing machine executable instructions. Examples of computer systems is a single computer, a network of computers, a cluster of computers, an embedded system, a mobile computing device such as a cellular telephone or personal digital assistant and a microcontroller. The machine executable instructions further comprise performing the step of detecting the gas bubble within the imaging volume using the magnetic resonance image.

In another embodiment the computer program product further comprises instructions for performing the step of creating a trend detected image by detecting contrast trends in the magnetic resonance image. The computer program product further comprises instructions for performing the step of creating a subtracted image by subtracting the magnetic resonance image from the trend detected image.

The computer program product further comprises in the step of creating a binary image by applying a threshold to the subtracted image. The computer program product further comprises performing the step of creating an output image by median filtering the binary image.

In another embodiment, the magnetic resonance image comprises a first plurality of pixels. The trend detected image comprises a second plurality of pixels. The subtracted image comprises a third plurality of pixels. The binary image comprises a fourth plurality of pixels. Each of the first plurality of pixels comprises a numerical value. Each of the second plurality of pixels comprises a numerical value. Each of the third plurality of pixels comprises a numerical value. For each of the first plurality of pixels there is a corresponding pixel in the first plurality of pixels, the second plurality of pixels, the third plurality of pixels, and the fourth plurality of pixels. The numerical value of each of the second plurality of pixels is calculated by detecting contrast trends in the first plurality of pixels. The numerical value of each of the third plurality of pixels is calculated by subtracting the corresponding pixel of the first plurality of pixels from the corresponding pixel of the second plurality of pixels or by subtracting the corresponding pixel of the second plurality of pixels from the corresponding pixel of the first plurality of pixels. Each pixel of the fourth plurality of pixels is assigned a first value if the corresponding pixel of the third plurality of pixels is above a predetermined threshold. Each of the fourth plurality of pixels is assigned a second value if the corresponding pixel of the third plurality of pixels is below the predetermined threshold.

When implemented by a computer, the first, second, third, and fourth plurality of pixels may be identical in some implementations. For instance when a plurality of pixels is not needed any longer it may be overwritten and reused to create another plurality of pixels. Another example is that when the binary image is created the third plurality of pixels may be overwritten with the binary image in order to create the fourth plurality of pixels.

In another embodiment the contrast trends are detected by performing the step of median filtering the magnetic resonance image to ensure that the numerical value of each of the plurality of pixels is within a predetermined range. The contrast trends are further detected by performing the step of low pass filtering the resulting median filtered image. Essentially the contrast trends are detected by first removing the extreme values from the pixels then applying a low pass filter which removes initially small details. What remains in the image after these two steps are large trends in the contrast of the image.

In another aspect the invention provides for a computer implemented method for detecting the gas bubble in magnetic resonance images. The method comprises the step of accessing a magnetic resonance image. The method further comprises creating a trend detected image by detecting contrast trends in the magnetic resonance image. The method further comprises creating a subtracted image by subtracting the magnetic resonance image from the trend detected image.

The method further comprises creating a binary image by applying a threshold to the subtracted image. The method further comprises the step of creating an output image by median filtering the binary image.

### BRIEF DESCRIPTION OF THE DRAWINGS

In the following preferred embodiments of the invention will be described, by way of example only, and with reference to the drawings in which:
Fig. 1 illustrates a magnetic resonance imaging system according to an embodiment of the invention;
Fig. 2 shows a block diagram illustrating a method according to an embodiment of the invention;
Fig. 3a shows a magnetic resonance image of a gel phantom with gas bubbles;
Fig. 3b shows an output image generated from Fig. 3a that identifies gas bubbles;
Fig. 4a shows a magnetic resonance image of a skin and ultrasound window interface; and
Fig. 4b shows an output image generated from Fig. 4a that identifies gas bubbles;

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

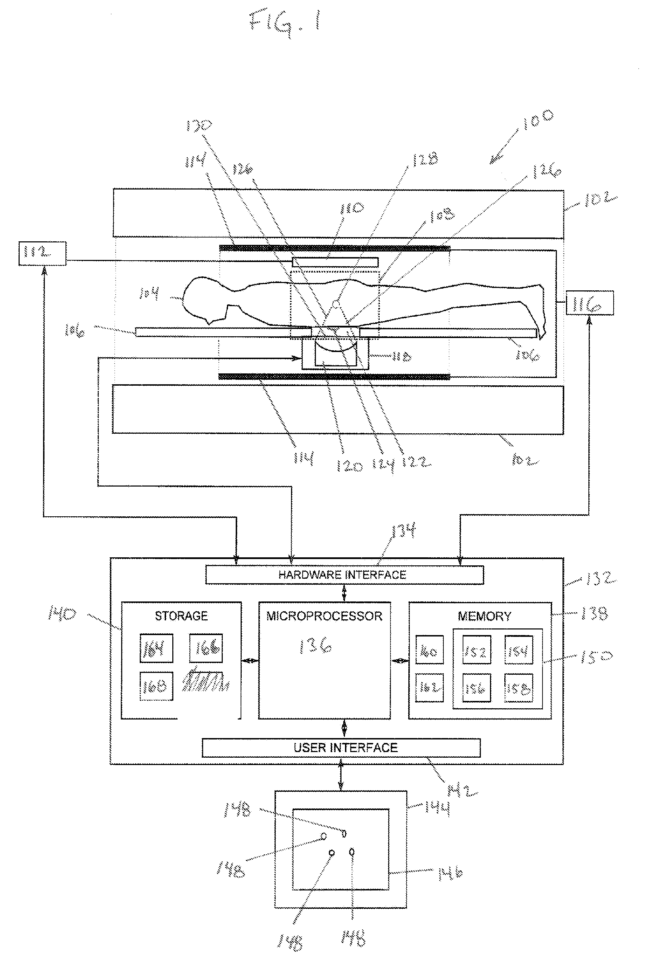

Fig. 1 shows a magnetic resonance imaging system 100 according to an embodiment of the invention. The magnetic resonance imaging system 100 has a magnet 102. In this example a cylindrical magnet with a bore through the center is shown. Other varieties of magnets could also be used. For instance an open style magnet which resembles two Helmholtz coils could also be used. Within the bore of the magnet 102 is located a subject 104 positioned on a subject support 106. The magnetic resonance imaging system 100 is adapted for acquiring magnetic resonance imaging data within an imaging zone 108. There is a radio frequency antenna 110 which acquires magnetic resonance imaging data within the imaging zone 108. The radio frequency coil 110 is connected to a radio frequency transceiver 112. Within the bore of the magnet is also a magnetic field gradient coil 114. The magnetic field gradient coil 114 is powered by a magnetic field gradient power supply 116.

Below the subject support 106 is an ultrasound system 118. In this example a focused ultrasound transceiver 120 of the type used in high intensity focused ultrasound systems is shown. The ultrasound system 118 has an ultrasound window 130 which is adapted for allowing ultrasonic waves to pass from the ultrasound system 118 into the subject 104. In this example there is a gap within the subject support 106 adapted for receiving a gel pad 122 which couples of the ultrasound window 130 to the subject 104. In this example a bubble 124 is shown between the gel pad 122 and the subject 104. However, the bubble 124 is located within the imaging zone 108. The volume through which ultrasound passes through the subject is bordered by the lines labeled 126. Ultrasound is focused in volume 128 within the subject. In this figure it can be seen that bubble 124 is within the boundaries 126 through which ultrasound passes. However, the bubble is within the imaging zone 108 and may be indentified within a magnetic resonance image 146.

The magnetic resonance imaging system 100 also comprises a computer system 132. The computer system has a hardware interface 134 which is adapted for connecting the computer system 132 to various other components of the magnetic resonance imaging system 100.

Radio frequency transceiver 112, the ultrasound system 118, and the magnetic field gradient power supply 116 are all shown as being connected to the hardware interface 134. Within the computer system 132 is a microprocessor 136. The microprocessor 136 is connected to the hardware interface 134, a user interface 142, computer storage 140 and computer memory 138. Microprocessor 136 is adapted for executing machine executable instructions. The computer memory 138 is adapted for storing data and machine executable instructions.

The computer memory stores a computer program product 150 which is adapted for controlling and running the magnetic resonance imaging system 100. The computer program product 150 comprises a magnetic resonance imaging system control module 152 which contains instructions for controlling and operating the magnetic resonance imaging system. The computer program product 150 further comprises an ultrasound control module 154 which contains machine executable instructions for controlling the operation of the ultrasound system 118. The computer program product 150 further comprises a magnetic resonance image construction module 156. This module contains routines and algorithms for constructing magnetic resonance images and renderings from magnetic resonance imaging data 160. The computer program product 150 further comprises a bubble identification module 158 which is adapted for performing image analysis of magnetic resonance images 162 for identifying gas bubbles 124. The memory 138 also has magnetic resonance imaging data 160 and a magnetic resonance image 162.

The computer system 132 also has computer storage 140. The computer storage may be in the form of a solid state hard drive, a conventional magnetic disc hard drive or other suitable computer storage medium. The computer storage 140 can store data and also copies of the computer program product 168. Within the computer storage is an archive of magnetic resonance imaging data 164 and also an archive of magnetic resonance images 166.

Also connected to the microprocessor 136 is a user interface 142. The user interface comprises elements that allow an operator to view data or information and also to control the operation of the computer system 132. For instance the user interface may comprise a mouse, a touchpad, a keyboard, or other user input device. Also shown in Fig. 1 is a display 144 which also comprises part of the user interface 142. On the display 144 is displayed a magnetic resonance image 146. Superimposed on the magnetic resonance image 146 are bubbles 148 which have been identified using the bubble identification module 158.

In operation a subject 104 will be placed on the subject support 106. The magnetic resonance imaging system 100 could be used to provide anatomical data which could be used for guiding the operation of the ultrasound system 118. Before initiating the sonication procedure the magnetic resonance imaging system acquires magnetic resonance imaging data in the region of the interface between the subject 104 and the gel pad 122. The bubble identification module 158 then identifies the gas bubble 124 between the subject 104 and the gel pad 122. An operator is notified of this using the display 144. The operator can then decide to move the subject 104 and remove the gas bubble 124 or to adjust the operation of the ultrasound transducer 120. This may involve either moving the ultrasound transducer 120 or controlling which elements of the ultrasound transducer are activated during the procedure.

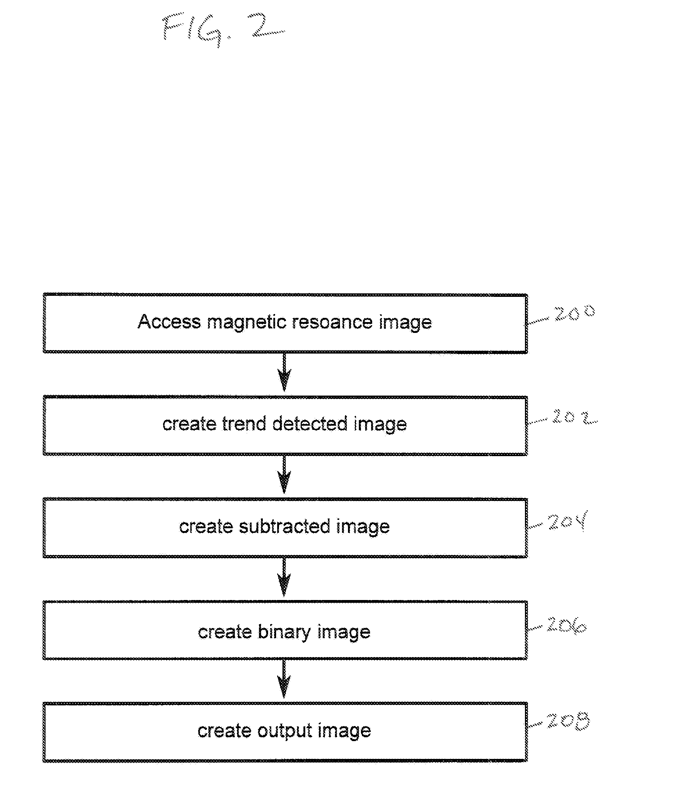

Fig. 2 shows an embodiment of a method according to an embodiment of the invention. In step 200 a magnetic resonance imaging image is accessed. A stored image may be accessed or a magnetic resonance image that has just been constructed from acquired magnetic resonance imaging data may be used. In step 202 an image in which large contrast trends are detected is created. In step 204 the magnetic resonance image is subtracted from the trend detected image. In step 206 a threshold is then applied to the subtracted image and finally in step 208 an output image is constructed. The output image is constructed by applying a median filter to the binary image.

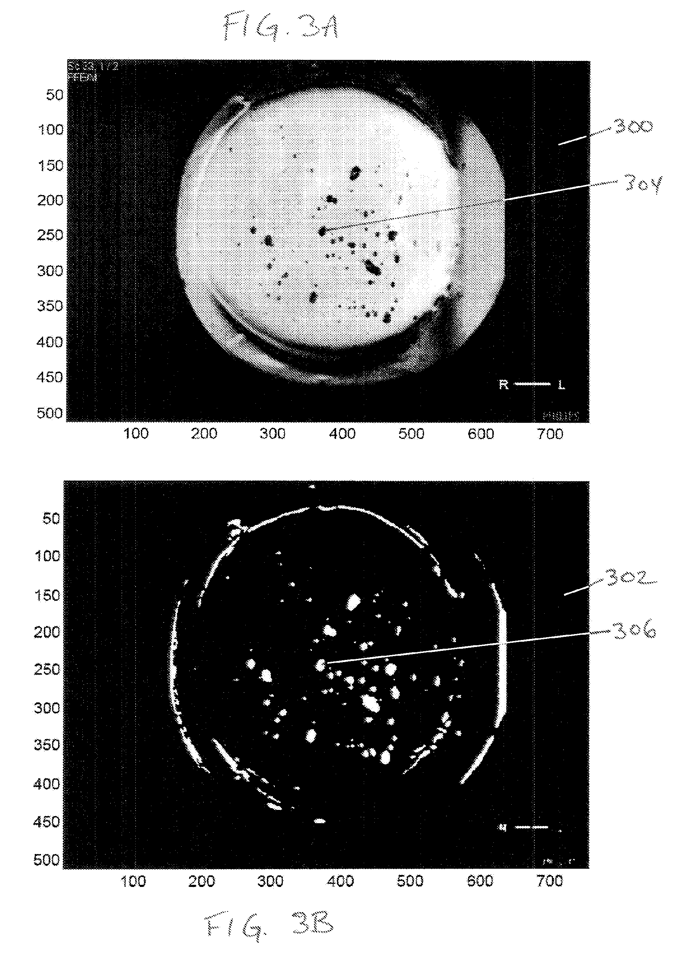

Fig. 3a shows a magnetic resonance image 300 of a gel phantom with gas bubbles embedded in it. Fig. 3b shows an output image 302 that has gas bubbles that have been identified in the image shown in Fig. 3a. The gas bubbles are identified in Fig. 3b as white regions in the image. For example bubble 300 is identified in Fig. 3a and a white region 302 corresponding to the bubble 300 is identified in Fig. 3b.

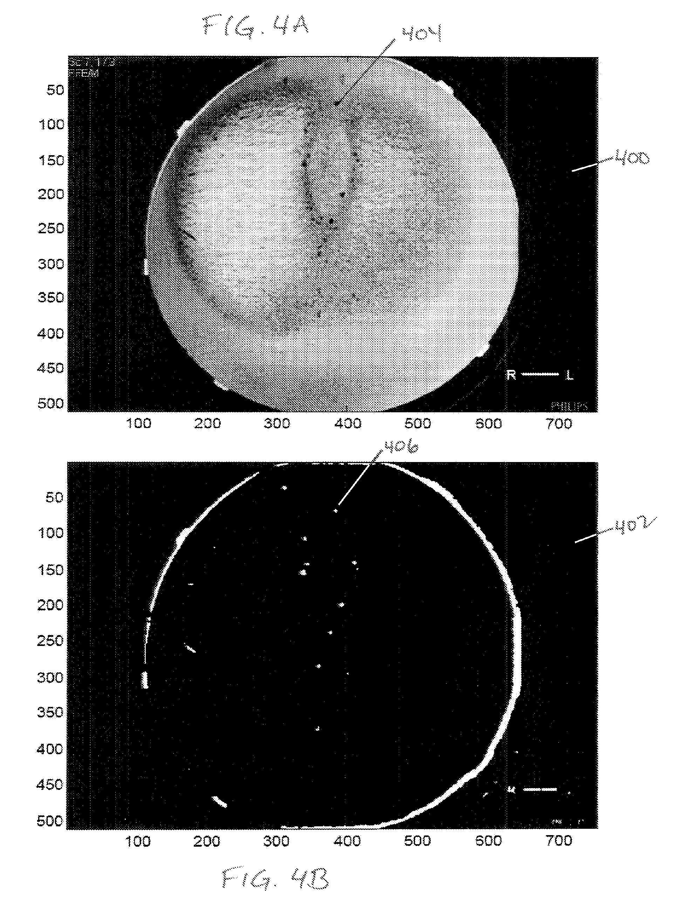

Fig. 4a shows a magnetic resonance image 400 of an interface between human skin and a gel pad. Gas bubbles in Fig. 4a are identified the output image 402 shown in Fig. 4b as being white regions. Figs. 4a and 4b demonstrate that embodiments of the method can be used for identifying gas bubbles between an ultrasound window and the surface of a subject. For example bubble 400 is identified in Fig. 4a and a white region 402 corresponding to the bubble 400 is identified in Fig. 4b.

### LIST OF REFERENCE NUMERALS:

- 100: Magnetic resoance imaging system
- 102: Magnet
- 104: Subject
- 106: Subject support
- 108: Imaging zone
- 110: Radio frequency coil
- 112: Radio frequency transciever
- 114: Magnetic field gradient coil
- 116: Magnetic field gradient coil power supply
- 118: Ultrasound system
- 120: Focued ultrasound transducer
- 122: Gel pad
- 124: Gas bubble
- 126: Path of ultrasound
- 128: Focus of ultrasound
- 130: Ultrasound window
- 132: Computer
- 134: Hardware interface
- 136: Microprocessor
- 138: Memory
- 140: storage
- 142: User interface
- 144: Display
- 146: Magnetic resonance image
- 148: Gas bubble
- 150: Computer program product
- 152: Magnetic resonance imaging system control module
- 154: Ultrasound system control module
- 156: Magnetic resoance image construction module
- 158: Bubble identification module
- 160: Magnet resoance imaging data
- 162: Magnet resoance image
- 164: Magnetic resoance imaging data archive
- 166: Magnetic resoance image archive
- 168: Computer program product
- 300: Magnetic resoance image
- 302: Output image
- 304: Gas bubble
- 306: White region
- 400: Magnetic resoance image
- 402: Output image
- 404: Gas bubble
- 406: White region

## Claims

1. A magnetic resonance imaging system (100) for detecting a gas bubble (124, 148, 304, 306, 404, 406) within an imaging volume (108), the magnetic resonance imaging system comprising:
- a magnet (102) adapted for generating a magnetic field for orientating the magnetic spins of nuclei of a subject (104) located within the imaging volume;
- a radio frequency system (110, 112) adapted for acquiring magnetic resonance data (160, 164), wherein the radio frequency system comprises a radio frequency transceiver (112) and a radio frequency coil (110);
- a magnetic field gradient coil (114) adapted for spatial encoding of the magnetic spins of nuclei within the imaging volume;
- a magnetic field gradient coil power supply (116) adapted for supplying current to the magnetic field gradient coil; and
- a computer system (132) adapted for constructing images from the magnetic resonance imaging data and for controlling the operation of the magnetic resonance imaging system, wherein the computer system is adapted for detecting the gas bubble within the imaging volume using a magnetic resonance image (162, 166, 300, 400) constructed from the magnetic resonance imaging data.

2. The magnetic resonance imaging system of Claim 1, wherein the computer system is adapted for detecting the gas bubble within the imaging volume by performing the steps of:
- accessing (200) the magnetic resonance image;
- creating (202) a trend detected image by detecting contrast trends in the magnetic resonance image;
- creating (204) a subtracted image by subtracting the magnetic resonance image from the trend detected image;
- creating (206) a binary image by applying a threshold to each of the third plurality of pixels; and
- creating (208) an output image (146, 302, 402) by meidan filtering the binary image.

3. The magnetic resonance imaging system of Claim 1 or 2, wherein the magnetic resonance imaging system is adapted for acquiring magnetic resonance imaging data with a gas bubble sensitive pulse sequence.

4. The magnetic resonance imaging system of Claim 3, wherein the gas bubble sensitive pulse sequence is a gradient echo sequence, wherein the gradient echo sequence has an echo time between 5 and 25 milliseconds, wherein the gradient echo sequence has a resolution of between 0.8 and 1.5 millimeters, wherein the gradient echo sequence has a slice thickness of between 1 and 4 millimeters, and wherein the gradient echo sequence has a flip angle between 10 and 80 degrees.

5. The magnetic resonance imaging system of any one of the preceding Claims, wherein the magnetic resonance imaging system further comprises an ultrasound system (118) for generating ultrasonic waves; wherein the ultrasound system comprises an ultrasound window (130); and wherein the ultrasound window is adapted for receiving the subj ect.

6. The magnetic resonance imaging system of Claim 5, wherein the magnetic resonance imaging system is adapted to detect the gas bubble between the ultrasound window and the subj ect.

7. The magnetic resonance imaging system of Claim 5 or 6, wherein the ultrasound system is a high intensity focused ultrasound system (118, 120, 130).

8. The magnetic resonance imaging system of any one of Claim 5, 6, or 7, wherein the computer system is further adapted for calculating a path (126) of the ultrasonic waves; wherein the computer system is further adapted for calculating the attenuation of the ultrasonic waves along the path by the gas bubble; and wherein the magnetic resonance imaging system is further adapted for signaling an operator if the attenuation of the ultrasonic waves is above a predetermined attenuation threshold.

9. The magnetic resonance imaging system of Claim 8, wherein the ultrasound system is adapted for adjusting the path of the ultrasound waves if the predetermined attenuation threshold is exceeded.

10. The magnetic resonance imaging system of any one of the preceding Claims, wherein the computer system comprises a display (144); and wherein the display is adapted for displaying the output image.

11. A computer program product for detecting a gas bubble (124, 148, 304, 306, 404, 406) within a magnetic resonance image (162, 166, 300, 400), the computer program product comprising machine executable instructions for execution by a computer system (132) for performing the following steps:
- accessing (200) a magnetic resonance image (162, 166, 300, 400) constructed from magnetic resonance imaging data (160, 164) of an imaging volume (108); and
- detecting the gas bubble within the imaging volume using the magnetic resonance image.

12. The computer program product of claim 11, wherein detection of the gas bubble within the imaging volume using the magnetic resonance image comprises the steps of:
- creating (202) a trend detected image by detecting contrast trends in the magnetic resonance image;
- creating (204) a subtracted image by subtracting the magnetic resonance image from the trend detected image;
- creating (206) a binary image by applying a threshold to each of the third plurality of pixels; and
- creating (208) an output image (146, 302, 402) by median filtering the binary image.

13. The computer program product of claim 12, wherein the magnetic resonance image comprises a first plurality of pixels; wherein the trend detected image comprises a second plurality of pixels; wherein the subtracted image comprises a third plurality of pixels; wherein the binary image comprises a fourth plurality of pixels; wherein each of the first plurality of pixels comprises a numerical value; wherein each of the second plurality of pixels comprises a numerical value; wherein each of the third plurality of pixels comprises a numerical value; wherein for each of the first plurality of pixels there is a corresponding pixel in the first plurality of pixels, the second plurality of pixels, the third plurality of pixels, and the fourth plurality of pixels; wherein the numerical value of each of the second plurality of pixels is calculated by detecting contrast trends in the first plurality of pixels; wherein the numerical value of each of the third plurality of pixels is calculated by subtracting the corresponding pixel of the first plurality of pixels from the corresponding pixel of the second plurality of pixels or by subtracting the corresponding pixel of the second plurality of pixels from the corresponding pixel of the first plurality of pixels; wherein each pixel of the fourth plurality of pixels is assigned a first value if the corresponding pixel of the third plurality of pixels is above a predetermined threshold; and wherein each of the fourth plurality of pixels is assigned a second value if the corresponding pixel of the third plurality of pixels is below the predetermined threshold.

14. The computer program product of Claim 11 or 12, wherein the contrast trends are detected by performing the steps of:
- median filtering the magnetic resonance image to ensure that the numerical value of each of the first plurality of pixels is within a predetermined range; and
- low pass filtering the resulting median filtered magnetic resonance image.

15. A computer implemented method for detecting a gas bubble (124, 148, 304, 306, 404, 406) in a magnetic resonance image (162, 166, 300, 400), the method comprising:
- accessing (200) a magnetic resonance image;
- creating (202) a trend detected image by detecting contrast trends in the magnetic resonance image;
- creating (204) a subtracted image by subtracting the magnetic resonance image from the trend detected image;
- creating (206) a binary image by applying a threshold to each of the third plurality of pixels; and
- creating (208) an output image (146, 302, 402) by median filtering the binary image.
